Europäisches Patentamt

European Patent Office

Office européen des brevets

(19)

(11) Publication number: **0 302 968**

**A1**

(12)

# EUROPEAN PATENT APPLICATION

(21) Application number: **87307060.1**

(22) Date of filing: **10.08.87**

(51) Int. Cl.4: **C12P 5/02**

(43) Date of publication of application:
**15.02.89 Bulletin 89/07**

(84) Designated Contracting States:
**AT BE CH DE ES FR GB GR IT LI LU NL SE**

(71) Applicant: **MICHIGAN BIOTECHNOLOGY INSTITUTE**
**3900 Collins Road P.O. Box 27609**
**Lansing Michigan 48909(US)**

(72) Inventor: **Chartrain, Michel**
**405 Palomino Lane**
**Madison Wisconsin 53705(US)**
Inventor: **Zeikus, Joseph Gregory**
**4239 Sand Ridge Road**
**Okemos Michigan 48864(US)**

(74) Representative: **Wynne-Jones, John Vaughan et al**
**Wynne-Jones, Lainé & James 22, Rodney Road**
**Cheltenham Gloucestershire GL50 1JJ(GB)**

(54) Starter cultures and method for biomethanation.

(57) Starter cultures for the anaerobic biomethanation of a lactose-containing substrate, such as whey, contain live, reproducible cells of Leuconstoc mesenteroides, Desulfovibrio vulgaris, Methanosar cinabarkeri, and Methanobacterium formicicum. The starter cultures can be either a defined starter culture consisting essentially of a mixture of substantially pure cultures of the organisms or an adapted sludge starter culture prepared by culturing a sewage digestor sludge containing the organisms on the substrate until the microorganisms have adapted to the environment of the substrate.

EP 0 302 968 A1

## STARTER CULTURES AND METHOD FOR BIOMETHANATION

Technical Field

The present invention relates to starter cultures containing live microorganisms. More particularly, it relates to starter cultures containing live bacteria for use in an anaerobic biomethanation process for the production of methane from biomass by-products.

Background Art

There are a number of industrial processes that have biomass by-products with high biological oxygen demands that can create disposal problems. For example, whey is an important by-product of the cheese industry that displays a biological oxygen demand of 40,000 to 60,000 mg/l and can cause serious pollution problems. The dry fraction of whey represents about 6% and is mainly lactose with trace amounts of protein, fat, and various ions including calcium. Ultrafiltration and reverse osmosis treatment of whey allow recovery of the valuable protein fraction, but the permeate fraction still contains most of lactose and the original biological oxygen demand. The anaerobic digestion of whey is a potentially more valuable pollution control treatment because it allows for the reduction of up to 95% in the biological oxygen demand; it produces cells and methane which can be used as fuel and it produces no polluting by-products.

Anaerobic digestion processes normally employ sludge from municipal waste treatment plants as the source of microorganisms for the digestor. However, when new digestors are seeded with sludge, a long lag phase occurs because it takes time for the microorganisms in the sludge to adapt to the composition of the environment. It is known to use defined and adapted starter cultures for various food processes in the dairy industry, however, there are few defined or adapted starter cultures available for industrial pollution control for aerobic waste treatment and there are none available for anaerobic biomethanation processes.

Starter cultures have been designed for the biomethanation of single specific molecules such as glucose, fructose, sucrose, cellulose, pectin, and choline. The composition of these defined co-cultures is based on artificial mixing of species isolated from diverse ecosystems.

It obviously would be advantageous to have starter cultures for the anaerobic biomethanation of a complex by-product, such as whey, that did not require a lag phase before producing useful amounts of methane.

The main objects of the present invention are to disclose starter cultures which are particularly useful for the anaerobic biomethanation of a lactose containing substrate, such as whey, and a method of anaerobic biomethanation employing those starter cultures.

One of the starter cultures of the present invention is a defined starter culture which comprises a mixture consisting essentially of live bacterial cells of substantially pure cultures of Leuconstoc mesenteroides, Desulfovibrio vulgaris, Methanobacterium formicicum and Methanosarcina barkeri in amounts which are effective for the biomethanation of the lactose containing substrate.

Another starter culture is an adapted sludge from a sewage digestor which contains a mixture of the bacteria of the above defined culture and associated organisms.

Both of the starter cultures may, if desired, also contain sufficient amounts of essential minerals, growth factors and nutrients to insure the initial growth of the cultures.

The method of the present invention is an improvement upon the anaerobic digestion of a lactose-containing substrate, such as whey, using sludge from a sewage digestor as the seed and it comprises subjecting the substrate to anaerobic biomethanation conditions using as the seed the starter cultures of the present invention or an inoculum prepared from those starter cultures.

In addition to the aforementioned objects, other objects and advantages of the present invention will be apparent to those skilled in the art from the following description of the preferred embodiment and the experimental work.

In the preferred embodiment of the present invention the defined starter culture consists essentially of enough living reproducible cells of substantially pure cultures of Leuconstoc mesenteroides strain DL 5 (DSM No. 20343), Desulfo vibrio vulgaris strain N l7 (DSM No. 644), Methanobacterium formicicum strain M 7 (DSM No. l3l2) and Methanosarcina barkeri strain MM 7 (DSM No. 800) to serve as an inoculum for the effective anaerobic biomethanation of whey. Of course, other strains of the above organisms that function in a similar manner may be used.

The adapted sludge starter culture of the present invention may contain in addition to the organisms of the defined starter culture the following organisms Clostridium butyricum, Klebsiella oxytoca, Clostridium propionicum, and Methanothrix soehngenii. The adapted sludge starter culture is preferably prepared by feeding a sludge obtained from a fixed-bed upflow digestor operated by the Societe Lyonnaise des Eaux, Le Pec, France on whey.

In the preferred method the starter cultures are used directly as an inoculum. However, it may be more economical to first culture the starter cultures in progressively larger batches on a lactose substrate until sufficient cell populations are obtained for the inoculation of a large digestor.

When it is intended to serve as the inoculum the defined starter culture of the present invention will generally contain the equivalent of about $10^{10}$ cells per ml. of the Leuconstoc mesenteroides; about $10^{10}$ cells per ml. of Desulfo vibrio vulgaris; about $10^9$ cells per ml. of Methanobacterium formicicum and about $10^9$ cells per ml. of Methanosarcina barkeri. When it is intended to prepare an inoculum the number of cells per ml. can be lower, but the microbial composition of the culture should be proportionally equivalent.

The defined starter culture of the present invention may also contain, in addition to the microorganisms, buffering agents and growth stimulating nutrients, such as preservatives, if desired.

The microorganisms of the defined starter culture possess different enzymatic activities and properties. For example, the Leuconstoc mesenteroides ferments the lactose to mostly lactate and smaller amounts of hydrogen, carbon dioxide, acetate and ethanol. Leuconstoc mesenteroides also was chosen for its ability to form exopolysaccharides useful in flocculation. The Desulfovibrio vulgaris ferments the lactate and ethanol to acetate, hydrogen and carbon dioxide. Desulfovibrio vulgaris also was selected for its high affinity towards and its syntrophic degradation of lactate and ethanol to acetate and hydrogen via methane dependent interspecies hydrogen transfer. The Methanobacterium formicicum ferments the $H_2O$ and $CO_2$ to methane and the Methanosarcina barkeri acetate to $CH_4$ and carbon dioxide to produce methane. Methanobacterium formicicum also was selected for its ability to flocculate and its hydrogen consumption kinetic properties. Methanosarcina barkeri also was selected for its superior umax/Ks ratio and its ability to flocculate.

The Clostridium butyricum and Klebsiella oxytoca of the adapted sludge starter culture are hydrolytic bacteria; the Clostridium propionicum is an acetogenic bacteria; and, the Methanothrix soehngenii is a methanogenic bacteria. The adapted sludge depending upon its source will also contain other organisms.

The microorganisms of the starter cultures possess the ability to grow together on a lactose substrate under anaerobic conditions, such as under a blanket of $N_2/CO_2$, at ambient temperatures (about 37° C) and neutral pHs (about 7). Furthermore, once in the preferred substrate, whey, the selected microorganisms do not generally require the addition of nutrients or growth factors for satisfactory results. Still further the use of the starter cultures of the present invention can result in a process in which over 95% of the lactose in whey can be converted in a 24 hour retention time; in contrast prior art processes required 5 to 13 days to achieve less complete conversions.

The method of the present invention utilizing the defined starter culture as the inoculum or source of the inoculum produces acceptable results on whey in an anaerobic digestor within about 12 days. The use of the adapted sludge inoculum usually takes about 16 days to reach comparable results. In contrast the use of sewage digestor sludge as a seed can take months and produce variable results. The difference in process time is due to the fact that the use of either the defined starter culture or the adapted sludge starter culture eliminates the lag phase that is required by the microorganisms in sewage digestor sludge to adapt to environmental conditions.

The present invention is further illustrated by the report of the experimental work which follows:

Experimental Procedures

The bacterial strains were isolated and cultured as described in the Chartrain et al. article in Applied and Environmental Microbiology Vol. 51, No. 1, pp. 188-196 (1986). The species used were: Leucon stoc mesenteroides strain DL 5, Desulfovibrio vulgaris strain N 17, Methanobacterium formicicum strain M 7, and Methanosarcina barkeri strain MM 7. Cells were grown in axenic cultures in 26 ml pressure tubes centrifuged for 15 min at 3000 × g, and resuspended in 2 ml of growth medium. A defined culture was initiated by inoculating a mixture of all species into 160 ml serum vials containing 50 ml of reduced Phosphate Buffered Basal (PBB) medium and 10 g/l of dry whey. Once lactose consumption and methane production were established, the defined mixed culture was transferred twice in bottles before being used to inoculate a chemostat containing PBB medium and 10 g/l of dry whey. Two bottles of defined culture were added to the chemostat which contained 160 ml of medium. After active methane production was detected in the culture, the peristaltic pump was turned on, and the retention time set to 100 h.

Simple chemostats were used as model anaerobic digestors and they contained 1 liter total capacity vessel with a 260 ml or 360 ml working volume. They were continuously fed by PBB medium containing 10 g/l of whey, or with raw whey, and were operated at a retention time of 100 h, 37°C, and pH 7.1.

Phase contrast and epifluorescense observations were performed with an Olympus microscope model BH2 equipped with a mercury lamp and an automatic exposure camera. The autofluorescence of the methanogenic bacterial factor $F_{420}$ was observed by using a B (IF-490) filter. Samples were taken from the chemostat by a syringe equipped with an 18 G 1 1/2 in. gauge needle and were placed on agar coated microscope slides, covered with a sealed coverslip and observed directly.

Carbon dioxide, hydrogen, acetate and ethanol were detected by standard gas chromatographic methods. Lactate was detected by high performance liquid chromatography. Lactose was measured by enzymatic analysis using a lactose assay kit. Dry weight was measured by filtration of a known volume on a 0.22 um porosity membrane, that was dried at 60°C to a constant weight. The m mole cell carbon was calculated using the following formula:

mg. cell dry weight $\times$ 0.451 mg. cell mg. C/12 mg. C.

The election composition of the cell was calculated using the value of 4.21 electron per mole of cell carbon.

A 10 ml sample was asseptically removed from the chemostats by syringe with an 18 G 1/2 in. needle and immediately introduced into a 26 ml sterile pressure tube containing one atmosphere of nitrogen, and 0.1 ml of a 2.5% $Na_2S$ solution. The cultures were centrigued at 3000 $\times$ g for 15 min. The supernatant was removed by syringe and 1 ml of a 20% sterile and anaerobic sucrose solution was added to the tubes as a carrier for freeze drying. The tubes were frozen in a mixture of dry ice and acetone. During the freezing process, the tubes were rolled in order to obtain a film on the inside walls of the tube. The tubes were asseptically connected to a Virtis freeze dryer through a connecting device developed for anaerobic cell preservation. After freeze drying, the tubes were pressurized with sterile nitrogen to prevent oxygen contamination. The tubes were kept at 4°C in a dessicator for long term storage.

The freeze dried starter cultures were rehydrated by the addition of 10 ml of sterile reduced PBB medium. Samples (3 ml) from the rehydrated culture tubes were inoculated into pressure tubes containing one atmosphere of nitrogen and 6 ml of reduced PBB medium containing 10 g/l for dry whey. These tubes were incubated at 37°C and both methane production and pH were monitored. If needed, the pH was corrected to near neutral by the addition of small amounts of sterile anaerobic 0.5 N NaOH solution. When active growth and methanogenesis was established, these tubes were used as seed for the chemostat.

The chemostat vessel was filled with 200 ml of non-sterile raw whey or PBB medium containing 10 g/l of dry whey, flushed with nitrogen for 10 min, 5 ml of 2.5% cysteine solution was added as reducing agent. The preserved cultures were reactivated as described above. Once methanogenesis resumed, five 10 ml tubes were added to the chemostat culture vessel. Once methane production was detected (less than 2 days), the peristaltic pump was turned-on and the retention time set for 100 h.

The defined starter culture strain composition was based on selecting the acetogenic, and methanogenic strains which displayed the best umax/Ks ratios. The selection of the lactose hydrolytic strain was made on the lactose fermentation end-products pattern. Experiments were then initiated to test the activity and stability of the defined culture for lactose biomethanation. In these experiments, the defined culture was comprised of species isolated from a non-defined sludge mixed flora which was adapted to whey. _Leuconstoc mesetenroides_ strain DL 5 was selected as the hydrolytic species and fermented lactose into mostly lactate, and smaller amounts of hydrogen, acetate, and ethanol. _Desulfovibrio vulgaris_ strain N 17 fermented lactate and ethanol into acetate, hydrogen, and carbon dioxide. _Methanobacterium formicicum_ strain M7 and _Methanosarcina barkeri_ strain MM7 were selected as the hydrogen-consuming and acetate-degrading methanogens respectively (Table 1). All four species were also present in the adapted sludge starter culture for whey biomethanation. All four species were readily distinguishable by microscopic observation of morphology.

Chemostats were seeded with either the defined culture or the adapted sludge starter culture and the biomethanation performance was compared at steady-state conditions in a defined medium (Table 2). The chemostat seeded with the defined culture took 12 days to reach steady state, while the digestor seeded with the adapted sludge took 16 days. The steady state concentrations of hydrogen and acetate were low and similar in both chemostats indicating a good performance. Both biomethanation systems produced mainly methane, carbon dioxide, and cells from lactose digestion. The defined starter culture system produced more cells and lower amounts of methane. The hydrolytic and acetogenic population levels were comparable in both systems, while the acetate-degrading methanogenic population was higher in the system seeded with the defined culture. However higher numbers may be an artifact caused by less clumping of _M. barkeri_ in defined culture because less methane was produced. The biomethanation performance and the carbon and electron recoveries were excellent in both digestor systems.

4

Microscopic examination of defined starter culture after growth in a steady state chemostat processing whey for l00 h retention time showed a population which was spacially organized both as free-living cells and self-immobilized cells in flocs. Cells of all species were present within flocs with the presence of methanogenic species being confirmed by epifluorescence microscopy.

Experiments were initiated to preserve population viability in the absence of metabolic activity on long term storage of either the defined culture or adapted sludge. Table 4 compares the relative population level of lactatedegrading bacteria and relative methanogenic activity of the defined cultures and adapted sludges that were stored for 6 months after lyophilization procedures. In whey biomethanation processes, lactose is hydrolysed primarily by oxygen tolerant organisms into lactate which is further sequentially fermented by oxygen sensitive acetogenic and methanogenic species. Thus, a prevalent oxygen sensitive trophic group was enumerated to represent culture viability and methanogenesis was quantified as a measure of metabolic activity. The freeze drying procedure was successful in preserving the viability and metabolic activity of both the defined culture and the adapted sludge.

The preserved defined culture was then used as a seed inoculum to start-up a chemostat for biomethanation of raw whey from a cheese manufacturing pilot plant. A long lag period was not required for either the defined starter culture or the adapted sludge starter culture to adapt to raw whey as substrate (i.e. l2 and l6 days, respectively). Table 3 shows the biomethanation performance of the defined starter culture processing raw whey in a chemostat at l00 h retention time. Steady state concentrations of metabolites were low, and most of the lactose was converted into methane, carbon dioxide, and cells. The biological oxygen demand removal was high reflecting good biomethanation performance.

It will be apparent from the results shown that both the defined starter culture and the adapted sludge starter culture were effective starter inoculums for the biomethanation of lactose in steady state chemostats using either sterile defined medium or raw whey directly. Furthermore, the starter cultures retained their viability and activity after preservation by freeze drying.

The nearly equivalent biomethanation performance displayed by chemostats inoculated with either the defined culture or the adapted sludge was also consistent with previous ecophysiological results on mixed and pure cultures. Namely, the species composition of the defined culture were comprised of members selected from adapted sludge flora that were the most competitive under the set of environmental conditions used in the chemostat. Their activity and stability is noteworthy because one might have anticipated the diverse population in adapted sludge may be required for cross-feeding of essential nutrients other than catabolic substrates.

TABLE 1.  Species composition of a defined culture for whey biomethanation

| Trophic group | Organisms | Principal substrate | End-products | Morphology |
|---|---|---|---|---|
| Hydrolytic | Leuconostoc mesenteroides | Lactose | Lactate Ethanol Acetate $H_2/CO_2$ | Coccus |
| $H_2$-producing acetogen | Desulfovibrio vulgaris | Lactate Ethanol | Acetate $H_2/CO_2$ | Vibrio |
| Methanogens | Methanosarcina barkeri | Acetate | $CH_4/CO_2$ | Sarcina |
| | Methanobacterium formicicum | $H_2/CO_2$ | $CH_4$ | Rod |

TABLE 2. Comparison of biomethanation performance of anaerobic digestors seeded with adapted sludge versus a defined culture.[a]

| Parameter | Adapted sludge | Defined culture |
|---|---|---|
| Lactose input/h (mmol) | 0.059 | 0.077 |
| $CO_2$ production/h (mmol) | 0.26 | 0.24 |
| $CH_4$ production/h (mmol) | 0.27 | 0.21 |
| Acetate steady state concentration (mM) | $0.660 \pm 0.300$ | $0.532 \pm 0.030$ |
| Hydrogen steady state concentration (uM) | $2.5 \pm 0.08$ | $3.11 \pm 1.94$ |
| Cells production/h (mmol) | 0.068 | 0.120 |
| Carbon recovery (%) | 87 | 75 |
| Electron recovery (%) | 90 | 100 |
| Lactose degrading population (per ml) | $3.4 \times 10^{10}$ | $1.65 \times 10^{10}$ |
| Lactate degrading population (per ml) | $1.9 \times 10^9$ | $1.43 \times 10^{10}$ |
| Hydrogen degrading population (per ml) | $6.2 \times 10^8$ | $3.5 \times 10^9$ |
| Acetate degrading population (per ml) | $5.6 \times 10^6$ | $6.8 \times 10^9$ |

a.     The chemostats were operating at 37 °C, pH 7.1, and fed with ?3B medium containing 10 g/l dry whey.

TABLE 3.     Steady state biomethanation performance parameters of a defined starter culture processing raw whey in a contact digestor at 100 h retention time.

| Parameter | Units |
|---|---|
| Lactose input/h (mmol) | 0.274 |
| $CH_4$ output/h (mmol) | 1.477 |
| $CO_2$ output/h (mmol) | 1.566 |
| Cells output/h (mmol) | 0.477 |
| Carbon recovery (%) | 107 |
| Electron recovery (%) | 105 |
| Acetate concentration (mM) | ≤1.00 |
| C.O.D. removal (%) | 88 |

a.   The chemostat was operating at 37 °C, pH 7.1, and fed with raw whey (36 g/l of lactose) at a 100 h retention time.

TABLE 4. Comparison of defined culture versus adapted
sludge preservation performance parameters.[a]

| Sample | Preservation parameters | |
|---|---|---|
| | Lactate-degrading population | Methane production |
| | MPN/ml | (umol/mg protein per tube) |
| Adapted sludge | $10^7$ | 9.09 |
| Defined culture | $10^5$ | 3.04 |

a. Experimental conditions: on adapted sludge or
defined culture, sample was removed from steady
state chemostats operated at 37 °C, pH 7.1, 100 h
retention time, and fed with PBB medium containing
10 g/l of dry whey. Samples were prepared in
anaerobic pressure tubes as described in methods.
After 6 months storage, treated samples were
rehydrated by addition of 10 ml of reduced PBB
medium containing 10 g/l dry whey, and the tubes
were incubated at 37 °C for 3 weeks. Results
represent average value for 3 preserved samples.

It will be readily apparent to those skilled in the art that other microorganisms can be incorporated in the starter cultures provided they do not detract from or interfere with the process. The incorporation of such additional microorganisms is therefore intended to be covered by the claims. In addition, other changes and modifications can be made without departing from the spirit and scope of the invention and such changes and modifications we also intended to be covered by the claims.

**Claims**

1. A starter culture for the anaerobic biomethanation of a lactose-containing substrate comprising a mixture of live, reproducible cells of Leuconstoc mesenteroides, Desulfovibrio vulgaris, Methanosarcinabarkeri, and Methano bacterium formicicum, said starter culture being selected from a defined starter culture consisting essentially of substantially pure cultures of said microorganisms and an adapted sludge starter culture containing said microorganisms which has been grown on the lactose-containing substrate so that the microorganisms are adapted to the environment of the substrate.

2. A defined starter culture for the anaerobic biomethanation of a lactose-containing substrate comprising a pure culture of a first microorganism that converts lactose to lactate and ethanol, a pure culture of a second microorganism that converts lactate and ethanol to acetate, hydrogen and carbon dioxide, a pure culture of a third microorganism which ferments the acetate to methane and carbon dioxide and a pure culture of a fourth microorganism that consumes hydrogen and carbon dioxide to produce methane.

3. A starter culture of claim 2 in which the first microorganism is Leuconstoc mesenteroides.

4. A starter culture of claim 2 in which the second microorganism is Desulfovibrio vulgaris.

5. A starter culture of claim 2 in which the third microorganism is Methanosarcina barkeri.

6. A starter culture of claim 2 in which the fourth microorganism is <u>Methanobacterium</u> <u>formicicum</u>.

7. A freeze dried starter culture for anaerobic biomethanation consisting of effective amounts of dormant but live reproducible cells of the microorganisms of claim I.

8. An adapted sludge starter culture for anaerobic biomethanation of lactose comprising a mixture of cells of <u>Leuconstoc</u> <u>mesenteroides</u>, <u>Desulfovibrio</u> <u>vulgaris</u>, <u>Methano</u> <u>bacterium</u> <u>formicicum</u> and <u>Methanosarcina</u> <u>barkeri</u>, said starter culture having been prepared by culturing a sewage digestor sludge containing said organisms on a substrate containing lactose.

9. A method for the anaerobic biomethanation of a lactose containing substrate which comprises adding to the substrate a starter culture of claim I which contains a mixture of living, reproducible cells of the microorganisms <u>Leuconstoc</u> <u>mesenteroides</u>, <u>Desulfovibrio</u> <u>vulgaris</u>, <u>Methano</u> <u>bacterium</u> <u>formicicum</u> and <u>Methanosarcina</u> <u>barkeri</u> or an inoculum prepared from said starter culture and culturing said microorganisms on said substrate under anaerobic conditions until a significant amount of the lactose has been consumed.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | APPLIED AND ENVIRONMENTAL MICROBIOLOGY, vol. 53, no. 5, May 1987, pages 1147-1156, American Society for Microbiology; M. CHARTRAIN et al.: "Microbial ecophysiology of whey biomethanation: comparison of carbon transformation parameters, species composition, and starter culture performance in continuous culture" * Whole document * ----- | 1-9 | C 12 P 5/02 |

TECHNICAL FIELDS SEARCHED (Int. Cl.4)

C 12 P

The present search report has been drawn up for all claims

| Place of search | Date of completion of the search | Examiner |
|---|---|---|
| THE HAGUE | 15-04-1988 | COUCKE A.O.M. |

EPO FORM 1503 03.82 (P0401)